# EUROPEAN PATENT APPLICATION

(11) **EP 1 980 612 A1**
(43) Date of publication of application: **15.10.2008**
(21) Application number: 07290438.6
(22) Date of filing: 10.04.2007
(51) Int. Cl.: C12N 1/20, C12P 7/62, A23L 1/00, A61K 8/00, A61K 31/00, C07C 67/00, C11D 3/00, C12R 1/01

(54) **Paraben compounds**

(71) Applicant: MUSEUM NATIONAL D'HISTOIRE NATURELLE, 75231 Paris Cedex 05 (FR); Centre National de la Recherche Scientifique, 75016 Paris (FR); Université Pierre et Marie Curie (Paris VI), 75252 Paris Cedex 05 (FR)
(72) Inventor: Bourguet-Kondracki, Marie-Lise, 92500 Rueil-Malmaison (FR); Domart-Coulon, Isabelle, 92370 Chaville (FR); Quévrain, Elodie, 77670 Vernou la Celle sur Seine (FR)
(74) Representative: Noel, Chantal Odile

(57) **Abstract**

The invention relates to a *Microbulbifer* bacterial strain isolated from a marine sponge, to its use in a process for manufacturing paraben compounds. The invention also relates to paraben compounds obtained from that *Microbulbifer* strain and to their uses.

The *Microbulbifer* strain of the invention is *Microbulbifer* strain named L4-N2 deposited at the CNCM under the registration number I-3714.

The process of manufacturing paraben compounds of the invention makes use of this *Microbulbifer* strain.

The invention finds application in the manufacture of paraben compounds, for use in particular in cosmetic, pharmaceutical, antifouling, detergent, sanitizing and disinfectant compositions.

## Description

The invention relates to a *Microbulbifer* bacterial strain isolated from a marine sponge and its use in a process for manufacturing paraben compounds.

It also relates to some paraben compounds and their uses.

Parahydroxybenzoic acid esters, parabens compounds, are well known antimicrobial preservatives allowed for use in food, drugs, cosmetics and toiletries.

They are generally used in combination containing two or more paraben compounds and/or preservatives.

Under EC Directive 95/2/EC, Annex III, methyl-, ethyl- and propyl parabens and their sodium salts are permitted for use, in particular, in a limited number of food products in combination with other preservatives. Under AFSSAPS evaluation (Commission de cosmétologie 29-09-2005 in Bulletin n°30, Dec. 2005, AFSSAPS Vigilance), methyl-ethyl-propyl-and butyl parabens are permitted for use in cosmetics.

Furthermore, the levels of paraben compounds in different products are limited by sanitary authorities of most countries.

Paraben compounds have also numerous other applications.

For example, they can be used as antimicrobial, antiseptic and/or antifouling agents. Therefore they can be used in numerous compositions such as in antifouling compositions, pesticide compositions, herbicidal compositions, in the production of antimicrobial nonwoven textiles and in antimicrobial acaricide compositions, aqueous inks, dental fillings and sealing compositions and make-up compositions.

Industrially, paraben compounds are presently issued from petrochemical processes. They are produced from parahydroxybenzoic acid, 4HBA, which is itself produced using some toxic solvents such as phenol.

Xue Peng et al., in Applied and Environmental Microbiology, August 2006, pp 5556-5561 reported that a strain of marine bacteria (strain A4B-17), belonging to the genus *Microbulbifer* and isolated from a tropical ascidian, is able to produce 4-hydroxybenzoate (4HBA) and its butyl-, heptyl- and nonyl esters. These authors tested 23 other strains belonging to the same genus *Microbulbifer,* and originating from various marine sources and geographic origins, mostly tropical. All these strains were able to produce some 4HBA, although in lower amounts than strain A4B-17 (less than 20%). However, none of them, but strain A4B-17, were able to produce paraben compounds.

The present invention is based on the discovery of another marine bacterial strain belonging to the genus *Microbulbifer* which is able to produce a family of paraben compounds, including in particular decyl- and dodecyl parabens. Indeed, this strain, hereinafter named "L4-N2", enables to obtain a broader variety of paraben compounds than strain A4B-17.

The strain L4-N2 has been deposited under the Budapest Treaty on January 24^{th} 2007, at the Collection Nationale de Cultures de Microorganismes (CNCM), 25 rue du Docteur Roux, Paris, France, under the registration number I-3714.

The strain L4-N2 is phylogenetically affiliated to the genus *Microbulbifer,* and clusters together with the species *Microbulbifer arenaceous* which was isolated from red sandstone off the coast of Scotland (Tanaka et al., Curr Microbiol, 47, 412, 2003), with which it shares 99.8% sequence identity on the basis of compared 16S rRNA sequences. Both *M arenaceous* and the L4-N2 strain belong to a different phylogenetic sub-group than strain A4B-17 and than the 4HBA producing *Microbulbifer* species described by Peng *et al.*

Bacteria of the strain L4-N2 are gram negative, aerobic, rod-shaped, and approximately 1 µm wide by 5 to 7 µm long. They form smooth, convex and mucoid colonies, and produce a non-diffusible brown pigment. They use glucose and display beta-galactosidase, catalase, and gelatinase activities. They are resistant to ampicillin, penicillin and oxacillin and sensitive to kanamycine (30UI) and ceftazidime (30µg).

They can be cultivated on classical media for marine bacteria, for instance those described by Zobell (J. Mar. Res. 4, 42,1941); examples of such media are DIFCO Marine Agar 2216 and Marine Broth 2216 (2.6 g/L NaCl).

Their growth has been tested at temperatures ranging from 8 to 30°C, and pH ranging of from 6.8 to 8.0. They can grow over this whole range of temperature and pH. The production of paraben compounds is however optimized when the bacteria are grown at temperatures ranging from 12°C to 20°C.

An object of the invention is the *Microbulbifer* sp. strain L4-N2 defined above.

A further object of the invention is the use of bacteria belonging to the genus *Microbulbifer,* and having, like the strain L4-N2 described above, the property to produce a non-diffusible brown pigment, for preparing paraben compounds, or as a starting material for purifying enzymes involved in the production of paraben compounds, or polynucleotides encoding said enzymes. Such bacteria can in particular be isolated from marine calcareous sponges, preferably temperate-water sponges, for instance sponges of the genus *Leuconia* and more specifically from the species *Leuconia nivea.* Preferably, their isolation includes a selective culture on medium supplemented with nalidixic acid. They can easily be identified on the basis of the brown pigmentation of their colonies.

According to a preferred embodiment of the invention, said bacteria belong to the *Microbulbifer arenaceous* species complex. Bacteria belonging to a same species complex are herein defined as bacteria having 16S rRNAs sharing at least 99% sequence identity.

According to a particularly preferred embodiment of the invention, said bacteria belong to the strain L4-N2 defined above.

The invention provides in particular a process for preparing paraben compounds, characterized in that it comprises culturing paraben compounds producing bacteria belonging to the genus *Microbulbifer,* as defined above, and recovering the paraben compounds from the culture. Preferably, said bacteria belong to the *Microbulbifer arenaceous* species complex, and still more preferably to the strain L4-N2.

Advantageously, said bacteria are grown at a temperature of about 12-20°C, and the paraben compounds are recovered when the culture is in the stationary phase (OD _{600 nm} value above 2), preferably within 12 to 24 hours after the culture has entered said stationary phase.

A mixture of paraben compounds can be easily recovered from the culture supernatant and/or the bacteria cells using any appropriate solvent, for instance CH₂Cl₂ or ethyl acetate (AcOEt).

This mixture of paraben compounds comprises in particular the compounds which are more specifically described hereinafter.

The individual paraben compounds can be isolated from this mixture using well known methods such as successive chromatographies of the CH₂Cl₂ extract on silica gel and reverse-phase columns.

The following nine compounds belonging to the paraben series are isolated by these methods.

**Compound 1:** 4-Hydroxy-benzoic acid ethyl ester, or ethylparaben, of the following formula 1:

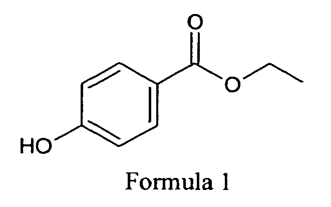

**Compound 2:** 4-Hydroxy-benzoic acid butyl ester, or butylparaben, of the following formula 2:

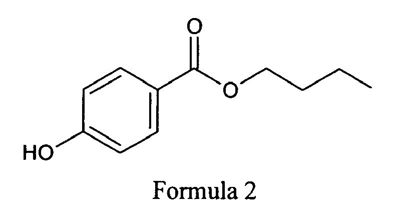

**Compound 3:** 4-Hydroxy-benzoic acid octyl ester, or octylparaben, of the following formula 3:

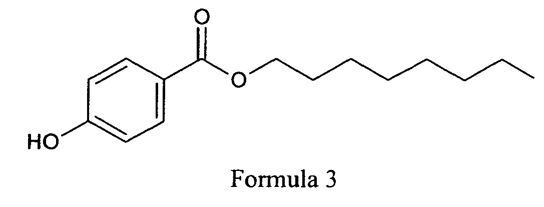

**Compound 4:** 4-Hydroxy-benzoic acid decyl ester, or decylparaben, of the following formula 4:

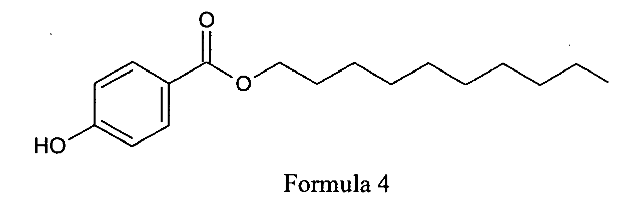

**Compound 5:** 4-Hydroxy-benzoic acid 3-hydroxy-decyl ester, or hydroxydecylparaben, of the following formula 5:

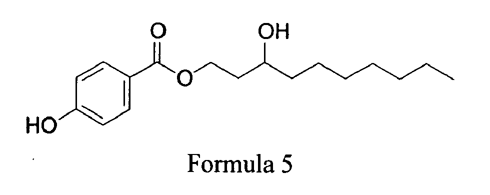

**Compound 6:** 4-Hydroxy-benzoic acid methyl-decyl ester, or methyldecylparaben, of the following formula 6:

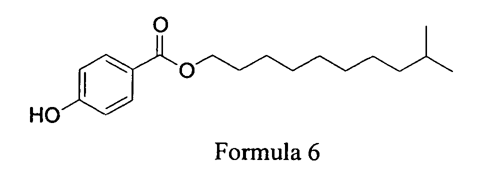

**Compound 7:** 4-Hydroxy-benzoic acid 3-hydroxy-methyl-decyl ester, or hydroxymethyldecylparaben, of the following formula 7:

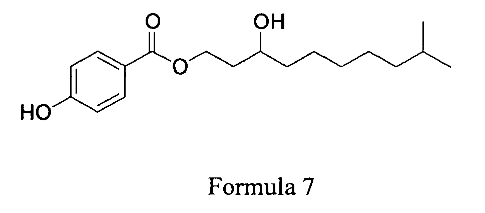

**Compound 8:** 4-Hydroxy-benzoic acid dodecyl ester, or dodecyl parabens, of the following formula 8:

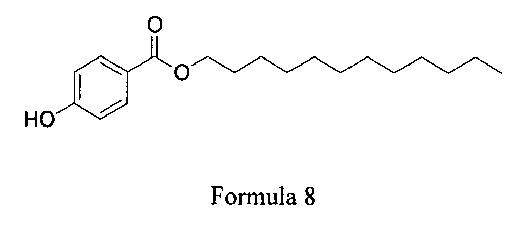

**Compound 9:** 4-Hydroxy-benzoic acid dodec-5-enyl ester of the following formula 9:

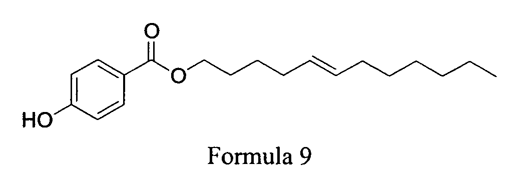

These purified paraben compounds isolated from the culture of the strain in the *M arenaceous* species complex surprisingly exhibited much higher *in vitro* activity than commercial synthetic methyl- and propyl- parabens usually used as antimicrobial preservative.

The invention will be better understood and other advantages and features thereof will appear more clearly when reading the following specification which refers to some examples which are given only for illustrative and non-limitative purposes.

### General experimental procedures

Optical rotations were obtained on a Perkin Elmer 341 polarimeter. UV spectra were obtained in EtOH, using a Kontron-type Uvikon 930 spectrophotometer, and IR spectra were recorded on a FTIR Shimatzu 8400S spectrophotometer.

Silica gel column chromatographies were carried out using Kieselgel 60 (230-400 mesh, E. Merck). Fractions were monitored by TLC using aluminium-backed sheets (Si gel 60 F254, 0.25 mm thick) with visualization under UV (254 and 366 nm) and Libermann spray reagent. Analytical reversed-phase HPLC (Kromasil KR5C18-25QS 250 x 4,6 mm column and C18 Uptisphere 250 x 7.8 mm column) were performed with a L-6200A pump (Merck-Hitachi) equipped with a UV-vis detector (λ = 254 nm) L-4250C (Merck-Hitachi) and a chromato-integrator D-2500 (Merck-Hitachi).

Mass spectra were recorded on an API Q-STAR PULSAR I of Applied Biosystem.

¹³C NMR spectra were obtained on a Bruker AC300 at 75.47 MHz, ¹H NMR spectra 1D and 2D (COSY, HSQC, HMBC, NOESY) were obtained on a Bruker AVANCE 400. HMQC and HMBC datas were acquired at 400.13 MHz using a ¹H -¹³C Dual probehead. The delay preceding the ¹³C pulse for the creation of multiple quanta coherences through several bounds in the HMBC was set to 70 ms.

### Biological material

Live specimens of the calcareous sponge *Leuconia nivea* Grant 1826(Porifera: Calcarea: Calcaronea: Baeriidae) were collected in the North-East Atlantic off the Bretagne Sud French coast in January 2006.

### Biological activity

Antimicrobial activity was tested by inhibition of growth of Gram positive bacteria *Staphylococcus aureus* strain ATCC 6538. All extracts and bacterial isolates from the sponge were screened for this antibacterial activity. Extracts and bacterial strains were also tested against gram negative gamma-proteobacteria (including *Vibrios*) and alpha-proteobacteria (Rhodobacterales), against Gram positive Bacillales (including *Bacillus sp.*) and against the yeast *Candida albicans.*

### Isolation of bacterial strains

One gram of fresh sponge was rinsed three times in filtered (0.2 µm) sea water and pressed through a 40 µm mesh-size plankton net in calcium and magnesium free artificial sea water. Serial dilutions 10⁻² and 10⁻⁴ of the maceration were spread onto Marine Agar supplemented with nalidixic acid (10 µg/mL) in order to increase the bacterial diversity recovered. All the plates were incubated at 15-20°C. After 14 days of incubation (5 days at 15 °C followed by 9 days at 4 °C), a brown pigmented convex colony was isolated from the 10⁻² dilution and propagated in pure culture on marine agar.

### Culture conditions

The bacterial strain was grown for 1 to 5 days in Marine Broth Difco 2216 (37.4 g/L) in the dark with rotary agitation at 350 rpm, at temperatures ranging from 8 to 30°C. The production of bioactive compounds was maximized at 15°C in pigmented culture arrested in stationary phase.

### Culture conditions and preparation of crude extract

A 25 ml inoculum, prepared from 5 colonies was grown in Marine Broth during 2 days at 15°C with shaking, then expanded to 2.5 L in 5L Erlenmayer flasks. Each bacterial culture was incubated for 2 days at 15°C with shaking under air. Cells were separated by centrifugation at 4°C during 15 min at 10,000 rpm (17,300 g). The supernatant was immediately poured in CH₂Cl₂ or AcOEt and incubated at least 12 hours (one night). The bacterial pellet was mechanically lyzed by 6 successive cycles of freeze-thaw plus sonication before to pouring in CH₂Cl₂ or AcOEt for extraction at least 12 hours (one night). In each case, the organic phase was concentrated in vacuum. Examination of Tlc revealed identical chemical profiles for CH₂Cl₂ or AcOEt extracts of both supernatant and pellet of the bacterial culture. Since they also showed identical antimicrobial activities, they were combined to constitute the CH₂Cl₂ or AcOEt crude extract of the culture. The CH₂Cl₂, extract was further extracted.

### Extraction and isolation

The CH₂Cl₂ extract (100 mg) was chromatographied on a silicagel (Merck silica gel 70-230 mesh) column using cyclohexane with increasing volume amounts of AcOEt as eluent. Bioassay-guided fractionation retained a maximum of activity in fractions eluted with 20% vol AcOEt/80% vol cyclohexane, fraction A and with 30% AcOEt/ 70% vol cyclohexane, fraction B

Fraction A eluted with 20% vol AcOEt/80% vol cyclohexane (17 mg) was subjected to reversed-phase HPLC column (C18 Uptisphere 250 x 7.8 mm) with increasing amounts of CH₃CN/H₂O as eluent (flow rate: 1 mL/min, wavelength: of 254 nm) to yield compound **1** (retention time: 9 min 79, 1.2 mg), compound **2** (retention time: 12 min 38, 1.0 mg), compound **4** (retention time: 30 min78, 1.2 mg), compound **6** (retention time: 33 min 68, 2.0 mg), compound **9** (retention time: 31 min 94, 0.8 mg).

In the same manner, fraction B eluted with 30% vol AcOEt/70% vol cyclohexane (4.6 mg) was subjected to reversed-phase HPLC (C18 Uptisphere 250 x 7.8 mm) using increasing amounts of CH₃CN/H₂O as eluent (flow rate: 1 mL/min, wavelength of 254 nm) to yield compound 5 (retention time: 6 min 54, 0.6 mg), compound 7 (retention time: 7 min 54, 0.6 mg).

Four bacterial cultures of 2.5 L were required to obtain enough quantities of pure compounds for structural elucidation and evaluation of antimicrobial activity.

The paraben compounds of the invention are obtained and isolated according to the above procedures.

### Example I: Identification of compound 1.

Ethylparaben, 4-Hydroxy-benzoic acid ethyl ester (0.64 mg per Liter of culture, 0.80 % of dry weight CH₂Cl₂ crude extract of the bacteria culture), white powder; mp 114-115°C; UV (EtOH) λₘₐₓ nm (ε): 286 (364) ; ¹H NMR CDCl₃ [δ_{H} in ppm] : 7.94 (2H, d, *J*= 8.7 Hz, H-2, H-6) ; 6.83 (2H, d, *J*= 8.7 Hz, H-3, H-5) ; 4.31 (2H, m, H-1') ; 1.35 (3H, t, *J*= 7.1 Hz, H-2'), ¹³C NMR: CDCl₃ (δ_{C} in ppm): 123.4 (C-1), 131.9 (C-2, C-6), 115.1 (C-3, C-5), 159.5 (C-4), 166.2 (C-7), 60.7 (C-1'), 14.4 (C-2'), ESI-MS [M+H]⁺ found at *m*/*z* 167.0708 (Δ -0.019 mmu), calc.167.0708 for C₉H₁₁O₃.

This compound has the following formula 1:

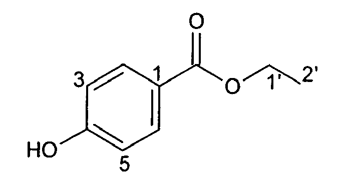

### Example II: Identification of compound 2.

Butylparaben, 4-Hydroxy-benzoic acid butyl ester (0.436 mg per Liter of culture, 1.78 % of dry weight CH₂Cl₂ crude extract of the bacteria culture), white powder; mp 68-69°C; UV (EtOH) λₘₐₓ nm (ε): 285 (575); ¹H NMR: CDCl₃ [δ_{H} in ppm] : 7.96 (2H, d, *J* = 9 Hz, H-2, H-6) ; 6.89 (2H, d, *J* = 9 Hz, H-3, H-5); 4.31 (2H, t, *J* = 6.5 Hz, H-1'); 1.75 (2H, m, H-2') ; 1.47 (2H, m, H-3'); 0.98 (3H, t, *J* = 7.3 Hz, H-4'); ¹³C NMR : CDCl₃ (δ_{C} in ppm): 122.7 (C-1), 131.9 (C-2, C-6), 115.2 (C-3, C-5), 160.2 (C-4), 167.0 (C-7), 64.7 (C-1'), 30.8 (C-2'), 19.3 (C-3'), 13.7 (C-4'), ESI-MS [M+H]⁺ found at *m*/*z* 195.1028 (Δ -0.6 mmu), calc.195.1093 for C₁₁H₁₅O₃.

This compound has the following formula 2:

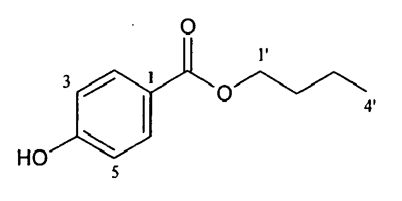

### Example III: Identification of compound 3.

Octylparaben, 4-Hydroxy-benzoic acid octyl ester (0.109 mg per Liter of culture, 0.43 % of dry weight CH₂Cl₂ crude extract of the bacteria culture), white amorphous solid; UV (EtOH) λₘₐₓ nm (ε): 283 (175); ¹H NMR: CDCl₃ [δ_{H} in ppm]: 7.94 (2H, d, *J* = 8.8 Hz, H-2, H-6); 6.83 (2H, d, *J* = 8.8 Hz, H-3, H-5), 4.25 (2H, t, *J* = 6.7 Hz, H-1'); 1.72 (2H, m, H-2'); 1.41 (2H, m, H-3'); 1.34 (2H, m, H-4'); 1.23 (2H, brs, H-5'); 1.27 (2H, m, H-6'); 1.26 (2H, m, H-7'); 0.86 (3H, t, *J* = 7.1 Hz, H-8'); ¹³C NMR: CDCl*₃* (δ_{C} in ppm): 124.2 (C-1), 132.3(C-2, C-6), 115.3 (C-3, C-5), 159.0 (C-4), 166.2 (C-7), 65.0 (C-1'), 28.6 (C-2'), 26.0 (C-3'), 29.1 (C-4'), 29.7 (C-5'), 31.8 (C-6'), 22.6 (C-7'), 13.9 (C-8'), ESI-MS [M+H]⁺ found at *m*/*z* 251.1652(Δ -0.48 mmu), calc.251.1647 for C₁₅H₂₃O₃.

This compound has the following formula 3:

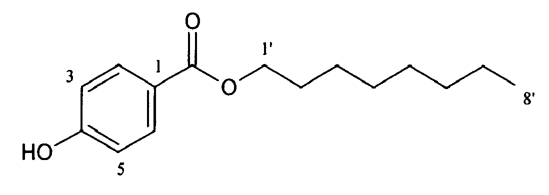

### Example IV: Identification of compound 4.

Decylparaben, 4-Hydroxy-benzoic acid decyl ester (0.254 mg per Liter of culture, 1.04 % of dry weight CH₂Cl₂ crude extract of the bacteria culture), pale yellow oil; UV (EtOH) λₘₐₓ nm (ε) : 285 (966); ¹H NMR: CDCl*₃* [δ_{H} in ppm] : 7.94 (2H, d, *J*= 8.8Hz, H-2, H-6) ; 6.83 (2H, d, *J*= 8.8 Hz, H-3, H-5) ; 4.26 (2H, t, *J*= 6.7 Hz, H-1') ; 1.73 (2H, m, H-2') 1.41 (2H, m, H-3'); 1.34 (2H, m, H-4'); 1.40 (2H, m, H-5'); 1.27 (4H, brs, H-6', H-7'); 1.24 (2H, m, H-8'), 1.26 (2H, m, H-9'); 0.86 (3H, t, *J*= 6.7 Hz, H-10') ; ¹³C NMR: CDCl*₃* (δ_{C} in ppm): 123.3 (C-1), 131.9 (C-2, C-6), 115.1 (C-3, C-5), 159.5 (C-4), 166.4 (C-7), 65.0 (C-1'), 28.7 (C-2'), 26.1 (C-3'), 29.3 (C-4'), 26.9 (C-5'), 29.5 (C-6'), 29.7 (C-7'), 31.9 (C-8'), 22.7 (C-9'), 14.1 (C-10'), ESI-MS [M+H]⁺ found at *m*/*z* 279.1962 (Δ -0.18 mmu), calc. 279.1960 for C₁₇H₂₇O₃.

This compound has the following formula 4:

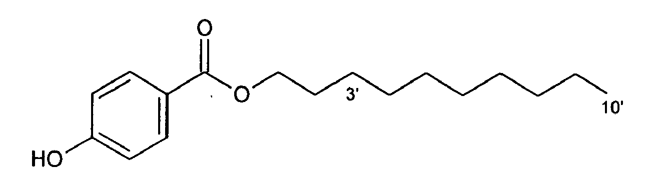

### Example V: Identification of compound 5.

Hydroxydecylparaben, 4-Hydroxy-benzoic acid 3-hydroxy-decyl ester (0.145 mg per Liter of culture, 0.59 % of dry weight CH₂Cl₂ crude extract of the bacteria culture), pale yellow oil, [α]_{D}²⁰= - 18 ° (*c* 0.14, MeOH), UV (EtOH) λₘₐₓ nm (ε): 286 (1378); ¹H NMR: CDCl*₃* [δ_{H} in ppm]: 7.93 (2H, d, *J*= 9 Hz, H-2, H-6) ; 6.83 (2H, d, *J*= 9 Hz, H-3, H-5); 4.58 (1H, m, H-1'a)-4.33 (1H, m, H-1'b); 1.92 (1H, m, H-2'a)-1.76(1H, m, H-2'b) ; 3.71 (1H, m, H-3'); 1.45 (2H, m, H-4'); 1.40 (1H, m, H-5'a)- 1.28 (1H, m, H-5'b) ; 1.26 (4H, brs, H-6', H-7') ; 1.23 (2H, m, H-8') ; 1.24 (2H, m, H-9') ; 0.84 (3H, t, *J*= 6.6 Hz, H-10'), ¹³C NMR: CDCl*₃* (δ_{C} in ppm): 123.3 (C-1), 131.8 (C-2, C-6), 115.2 (C-3, C-5), 159.6 (C-4), 168.1 (C-7), 62.0 (C-1'), 36.5 (C-2'), 68.6 (C-3'), 37.8 (C-4'), 25.5 (C-5'), 29.6* (C-6'), 29.5* (C-7'), 31.6 (C-8'), 22.9 (C-9'), 14.0 (C-10') ESI-MS [M+H]⁺ found at *m*/*z* 295.1913 (Δ -0.4 mmu), calc.295.1923 for C₁₇H₂₇O₄.
* may be interchanged

This compound has the following formula 5:

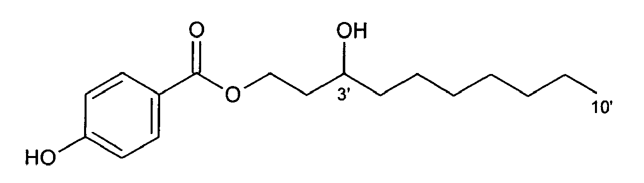

### Example VI: Identification of compound 6.

Methyldecylparaben, 4-Hydroxy-benzoic acid methyl-decyl ester (0.509 mg per Liter of culture, 2.08 % of dry weight CH₂Cl₂ crude extract of the bacteria culture),pale yellow oil; UV (EtOH) λₘₐₓ nm (ε): 286 (687); ¹H NMR: CDCl₃ [δ_{H} in ppm] : 7.92 (2H, d, *J=* 9 Hz, H-2, H-6) ; 6.82 (2H, d, *J*= 9 Hz, H-3, H-5); 4.26 (2H, t, *J*= 6.6 Hz, H-1') ; 1.72 (2H, m, H-2') ; 1.40 (2H, m, H-3') ; 1.30 (2H, m, H-4') ; 1.25 (2H, m, H-5') ; 1.31 (2H, m, H-6') ; 1.28 (2H, m, H-7') ; 1.13 (2H, m, H-8') ; 1.53 (1H, m, H-9') ; 0.88 (6H, d, *J*= 6.6 Hz, H-10', H-11'), ¹³C NMR: CDCl*₃* (δ_{C} in ppm) : 123.0 (C-1), 131.9 (C-2, C-6), 115.1 (C-3, C-5), 159.6 (C-4), 166.5 (C-7), 65.0 (C-1'), 28.7 (C-2'), 26.1 (C-3'), 29.5 (C-4'), 29.1 (C-5'), 29.7 (C-6'), 27.3 (C-7'), 39.0 (C-8'), 27.9 (C-9'), 22.6 (C-10', C-11'), , ESI-MS [M+H]⁺ found at *m*/*z* 293.2115 (Δ -0.07 mmu), calc. 293.2116 for C₁₈H₂₉O₃.

This compound has the following formula 6:

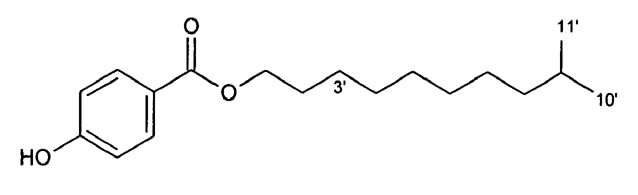

### Example VII: Identification of compound 7.

Hydroxymethyldecylparaben, 4-Hydroxy-benzoic acid 3-hydroxy-methyl-decyl ester (0.218 mg per Liter of culture, 0.89 % of dry weight CH₂Cl₂ crude extract of the bacteria culture), white amorphous solid; [α]_{D}²⁰ = - 7 ° (*c* 0.10, MeOH); UV (EtOH) λₘₐₓ nm (ε):287 (2000); ¹H NMR: CDCl*₃* [δ_{H} in ppm]: 7.92 (2H, d, *J*= 8.7 Hz, H-2, H-6) ; 6.83 (2H, d, *J*= 8.7 Hz, H-3, H-5); 4.57 (1H, m, H-1'a)-4.33 (1H, m, H-1'b) ; 1.93 (1H, m, H-2'a)-1.76(1H, m, H-2'b) ; 3.72 (1H, m, H-3') ; 1.47 (2H, m, H-4') ; 1.46 (1H, m, H-5'a)-1.31 (1H, m, H-5'b) ; 1.27 (2H, m, H-6') ; 1.25 (2H, m, H-7') ; 1.17 (2H, m, H-8') ; 1,26 (1H, m, H-9') ; 0.83 (6H, d, *J*= 6.6 Hz (H-10', H-11'), ¹³C NMR: CDCl*₃* (δ_{C} in ppm) : 122.7 (C-1), 131.9 (C-2, C-6), 115.1 (C-3, C-5), 160.2 (C-4), 166.7 (C-7), 61.9 (C-1'), 36.6 (C-2'), 68.8 (C-3'), 37.4 (C-4'), 25.4 (C-5'), 31.4 (C-6'), 29.4 (C-7'), 39.0 (C-8'), 27.9 (C-9'), 22.6 (C-10', C-11') ESI-MS [M+H]⁺ found at *m*/*z* 309.2055 (Δ -0.5 mmu), calc.309.2000 for C₁₈H₂₉O₄.

This compound has the following formula 7:

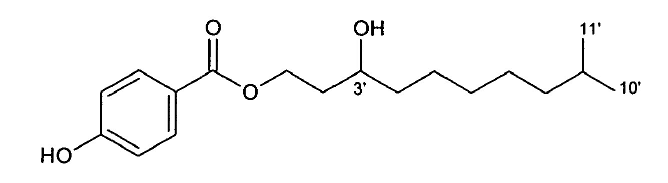

### Example VIII: Identification of compound 8.

Dodecylparaben, 4-Hydroxy-benzoic acid dodecyl ester (0.109 mg per Liter of culture, 0.43% of dry weight CH₂Cl₂ crude extract of the bacteria culture), white amorphous solid, UV (EtOH) λₘₐₓ nm (ε): 285 (966); ¹H NMR: CDCl*₃* [δ_{H} in ppm] 7.94 (2H, d, *J*= 8.8 Hz, H-2, H-6) ; 6.83 (2H, d, *J*= 8.8 Hz, H-3, H-5); 4.26 (2H, t, *J*= 6.7 Hz, H-1') ; 1.72 (2H, m, H-2'); 1.39 (2H, m, H-3'); 1.33-1.24 (12H, brs, H-4'-H-9'), 1,24 (2H, m, H-10') ; 1,23 (2H, m, H-11'), 0.86 (3H, t, *J*= 6.6 Hz (H-12'): , ¹³C NMR: CDCl*₃* (δ_{C} in ppm) :123.7 (C-1), 131.9 (C-2, C-6), 115.1 (C-3, C-5), 159,1 (C-4), 166.0 (C-7), 64.6 (C-1'), 28.7 (C-2'), 26.0 (C-3'), 29.6-29.0 (C-4'-C-9'), 31.9 (C-10'), 22.7 (C-11'), 14.1 (C-12'); ESI-MS [M+H]⁺ found at *m*/*z* 306.2278 (Δ -0.4 mmu), calc. 306.2273 for C₁₉H₃₁O₃.

This compound has the following formula 8:

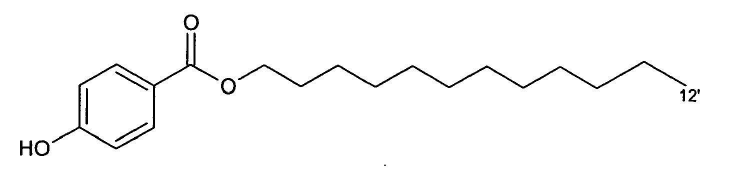

### Example IX: Identification of compound 9.

4-Hydroxy-benzoic acid dodec-5-enyl ester (0.145 mg per Liter of culture, 0.59 % of dry weight CH₂Cl₂ crude extract of the bacteria culture), white amorphous solid, UV (EtOH) λₘₐₓ nm (ε): 285 (334); ¹H NMR: CDCl*₃* [δ_{H} in ppm] : 7.94 (2H, d, *J*= 8.8 Hz, H-2, H-6) ; 6.83 (2H, d, *J*= 8.8 Hz, H-3, H-5); 4.27 (2H, t, *J*= 6.6 Hz, H-1'); 1.74 (2H, m, H-2') ; 1.47 (2H, m, H-3') ; 2.10 (2H, m, H-4') ; 5.35 (2H, m, H-5', H-6') ; 2.00 (2H, m, H-7'); 1.24 (4H, brs, H-8', H-9') ; 1.22 (2H, m, H-10'); 1.27 (2H, m, H-11'); 0.86 (3H, t, *J*= 6.7 Hz, H-12'), ¹³C NMR: CDCl*₃* (δ_{C} in ppm) : 123.2 (C-1), 131.9 (C-2, C-6), 115.1 (C-3, C-5), 159.5 (C-4), 166.2 (C-7), 64.7 (C-1'), 28.2 (C-2'), 26.1 (C-3'), 26.7 (C-4'), 129.2 (C-5'), 130.6 (C-6'), 27.2 (C-7'), 29.6 (C-8'), 29.0 (C-9'), 31.9 (C-10'), 22.5 (C-11'), 14.1 (C-12'), ESI-MS [M+H]⁺ found at *m*/*z* 305.2102 (Δ -1.4 mmu), calc.305.2116 for C₁₉H₂₉O₃.

This compound has the following formula 9:

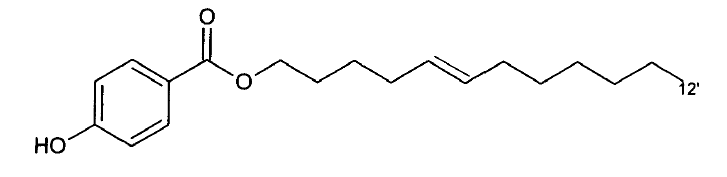

Among these nine products, compounds 1, 3, 4 and 8 are reported for the first time as natural products obtained from natural sources.

As to compounds 5, 6, 7, and 9, they are novel structures and were not reported before the invention.

### Determination of the Minimal Inhibitory Concentration (MIC) and of the Minimal Bactericidal Concentration (MBC).

MICs of each of the compounds 1-9 according to the invention and of commercial methyl- and propyl parabens of the prior art were determined by a twofold dilution method from an initial 100 µg/mL concentration. Hence, each compound was tested in the range 100 µg/mL through 0.048 µg/mL. Microplates were incubated for 24 hours at 30°C with shaking (500 rpm) and optical density at 600 nm was read to estimate bacterial growth. The methyl- and propyl parabens of the prior art were obtained from the company Interchimie in Bobigny, France.

MBCs were determined to estimate bacterial survival in wells with no visible growth after 24 h by subculturing onto agar plates to count the number of colony-forming-units (CFU/mL). The MBC was defined as the lowest concentration of compound yielding colony counts less than 0.1% of the initial inoculum.

The antimicrobial activity of the paraben compounds of the invention as well as the antimicrobial activity of the commercial methylparaben and propylparaben were confirmed towards *Staphylococcus aureus* (gram+) bacterial strain.

The results of this evaluation are summarized in the following table 1.

**Table 1: MICs and MBCs against Staphylococcus aureus (ATCC 6538) of pure natural compounds 1-9 and of crude and successive fractions of the CH₂Cl₂, extract. Commercial synthetic methyl- and propyl parabens were used as reference**

| Compounds | MIC (µM)* | MIC (µg/mL)* | MBC (µg/mL)* |
|---|---|---|---|
| Methyl paraben | 328.9 - 657.8 | 50 - 100 | > 100 |
| Propyl paraben | 277.7 - 555.5 | 50 - 100 | > 100 |
| **Crude extract CH₂Cl₂** | - | 25-50 | ND |
| **Fraction A** | - | 25-50 | ND |
| **Fraction B** | - | 50-100 | ND |
| **1** | > 602.0 | > 100 | > 100 |
| **2** | 128.8 - 257.7 | 25 - 50 | > 100 |
| **3** | 100 - 200 | 25 - 50 | 50 - 100 |
| **4** | 2.8 - 5.6 | 0.78 - 1.56 | 12.5 - 25 |
| **5** | 85 - 170 | 25 - 50 | > 100 |
| **6** | 42.8 - 85.6 | 12.5 - 25 | 25 - 50 |
| **7** | 40.5 - 81.1 | 12.5 - 25 | 25 - 50 |
| **8** | 81.6 - 163.3 | 25 - 50 | > 100 |
| **9** | 20.5 - 41.1 | 6.25 - 12.5 | 50 - 100 |

| | | | |
|---|---|---|---|
| • * : compounds were tested in triplicate in microtiter plates • ND: Not Determined MIC (Minimal Inhibitory Concentration) was expressed as the interval *a-b,* where *a* is the highest concentration tested at which microorganisms are growing and *b* the lowest concentration that causes 100% growth inhibition. MBC (Minimal Bactericidal Concentration) was defined as the lowest concentration of compound yielding colony counts < 0.1% of the initial inoculum. | | | |

As it can be seen from table 1, all of the paraben compounds isolated from the culture of *Microbulbifer sp.* L4-N2 strain exhibited strong antimicrobial activity against *S. aureus.* Compound **4** exhibited the greatest efficiency with MIC values of 2.8-5.6 µM. The length of the alkyl chain seems to increase considerably activity. We found that compounds **3, 4, 6, 8**, which bear respectively a C-8, C-10, C-11 and C-12 alkyl chain, had a higher potency and were about 2 to 100 fold more active than methyl and propyl synthetic parabens of the prior art as well as our natural, bacteria-derived ethyl **(1)** and butyl (2) parabens. Adding an unsaturation to the alkyl chain also enhances by a factor 4 the antimicrobial activity as illustrated by compared MIC values of compounds **8** (81.6 - 163.3µM) and **9** (20.5 - 41.1 µM). The presence of an hydroxyl functionality on the alkyl chain has little effect upon antimicrobial activity.

In order to determine whether the growth inhibition observed against *S*. *aureus* was relevant either of a bactericidal or a bacteriostatic effect, the ratio MBC/MIC was calculated. If MBC/MIC is less than 4, the compound is bactericidal, if MBC/MIC is between 8 and 16 and above 16, the compound is bacteriostatic. The results are summarized in the following table 2.

As it can be seen from that table 2, using this method, compounds 4 and 9 appeared as bacteriostatic compounds and compounds **3, 6** and **7** appeared as bactericidal ones.

**Table 2: MBC/MIC ratios.**

| Compounds | MBC/MIC ratio |
|---|---|
| Methyl paraben | ND |
| Propyl paraben | ND |
| **1** | > 16 |
| **2** | > 16 |
| **3** | 2 = bactericidal |
| **4** | 16 = bacteriostatic |
| **5** | > 16 |
| **6** | 2 = bactericidal |
| **7** | 2 = bactericidal |
| **8** | > 16 |
| **9** | 8 = bacteriostatic |

| | |
|---|---|
| N.D: Not determined. | |

Thus, compounds 3, 6, and 7 can be used as bactericidal agents, in particular in sanitizing and antiseptic compositions.

Such sanitizing compositions can be applied to floors, medical instruments and panels etc....in hospitals for killing unwanted bacteria.

They can also be used for sanitizing floors, panels etc....in home care products. Otherwise stated they can be used in detergent compositions.

We conclude that natural paraben compounds **3-9** have a better *in vitro* activity than synthetic methyl- and propyl parabens of the prior art and than natural ethyl- and butyl parabens **1-2**, obtained by the process of the invention, which are the four paraben compounds usually used as antimicrobial preservative.

The study of antimicrobial activities was pursued by searching a possible synergistic mode of action. Antimicrobial assays were simultaneously performed with mixture of compounds in an equimolar ratio.

The results are reported in the following table 3 in which the MIC is reported in µg/ml and expressed, as in table 1, as the interval *a-b,* where *a* is the highest concentration tested at which microorganism are growing and *b* the lowest concentration that causes 100% growth inhibition.

**Table 3: MIC (µg/mL) of different equimolar mixtures of paraben compounds of the invention.**

| | **Compound of formula 1** | **Compound of formula 2** | **Compound of formula 3** | **Compound of formula 4** | **Compound of formula 5** | **Compound of formula 6** | **Compound of formula 7** | **Compound of formula 8 of** | **Compound formula 9** |
|---|---|---|---|---|---|---|---|---|---|
| **Compound of formula 1** | | | | | | | | | |
| **Compound of formula 2** | 100 - 200 | | | | | | | | |
| **Compound of formula 3** | 50-100 | 25 - 50 | | | | | | | |
| **Compound of formula 4** | 6.25 - 12.5 | 3.12 - 6.25 | 25 - 50 | | | | | | |
| **Compound of formula 5** | 50 - 100 | 50 - 100 | N. D. | 6.25 - 12.5 | | | | | |
| **Compound of formula 6** | 25 - 50 | 12.5 - 25 | 3.12 - 6.25 | 3.12 - 6.25 | 25 - 50 | | | | |
| **Compound of formula 7** | 12.5 - 25 | 12.5 - 25 | N. D. | 6.25 - 12.5 | N. D. | 12.5 - 25 | | | |
| **Compound of formula 8** | 50 - 100 | 25 - 50 | N. D. | N. D. | N. D. | 6.25 - 12.5 | N. D. | | |
| **Compound of formula 9** | 12.5 - 25 | 12.5 - 25 | N. D. | 3.12 - 6.25 | N. D. | 12.5 - 25 | N. D. | N. D. | |

| | | | | | | | | | |
|---|---|---|---|---|---|---|---|---|---|
| N.D.: not determined. | | | | | | | | | |

One can see from table 3 that when two paraben compounds according to the invention are mixed together, the MIC of the combined compounds is always close to the best MIC between the two compounds. Equimolar combinations of either compounds 2 and 4, 3 and 6, 4 and 6 and 4 and 9 have a MIC of about 3-6 µg/mL

Table 3 also shows that when either the natural ethyl paraben or the natural butyl paraben manufactured according to the process of the invention is mixed with another natural paraben compound of the invention, the MIC of that mixture is very lower than the MIC of the natural ethyl- or butyl parabens alone, so that the quantity necessary to inhibit bacterial growth with the mixture is lowered as compared to the quantity of ethyl- or butyl paraben alone necessary to inhibit bacterial growth.

Furthermore, the MIC of the CH₂Cl₂ crude extract and the MIC of the fraction eluted with 20 % AcOEt are of 50 > MIC > 25µg/mL and the MIC of the fraction eluted with 30 % AcOEt (ethyl acetate) is of 100 > MIC > 50 µg/mL, which means that even the mixtures as obtained, i.e. before isolation of each paraben compounds, have excellent preservative properties, about 2 fold higher than the commercial synthetic methyl- and propyl parabens usually used as preservatives. So, the culture of strain L4-N2 can be used without isolation of each paraben compound, and based on its native combination of natural paraben compounds.

The CH₂Cl₂ crude extract also showed to be active against Gram negative strains (*Vibrio* gamma-proteobacteria and Rhodobacterales alpha-proteobacteria) as opposed to commercial paraben compounds which are not active against Gram-negative bacteria.

The antimicrobial activities of the sponge derived bacteria may contribute to the chemical defence strategy of the sponge host, for example against opportunistic environmental pathogens. They may also contribute to the balance of the microbial community structure, and the maintenance of its diversity, characteristic of sponges.

Due to their exceptional activity, the natural paraben compounds of the invention or the paraben compounds obtained by the process of the invention are particularly useful as preservative to be substituted to the commercial paraben compounds.

Moreover, due to their higher activities, the paraben compounds of the invention may be used in lower concentrations as compared to paraben compounds of the prior art while maintaining the same preservative properties, which is of great interest in view of the national regulations limiting the levels of paraben compounds in particular in food, cosmetic, pharmaceutical and toiletry compositions.

Thus, the invention also encompasses:
- a paraben compound having the following formula 5: 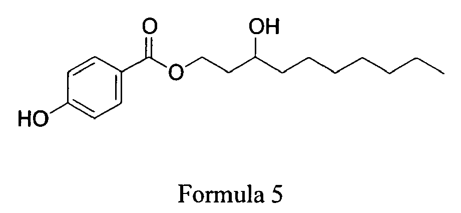 which is the 4-Hydroxy-benzoic acid 3-hydroxy-decyl ester,
- a paraben compound having the following formula 6: 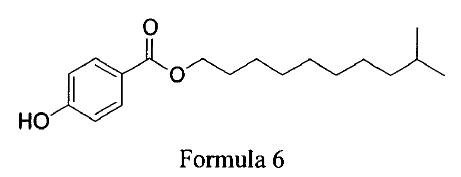 which is the 4-Hydroxy-benzoic acid methyl-decyl ester,
- a paraben compound having the following formula 7: 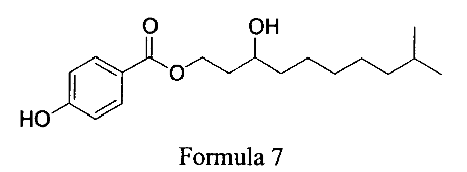 which is the 4-Hydroxy-benzoic acid 3-hydroxy-methyl-decyl ester, and
- a paraben compound having the following formula 9: 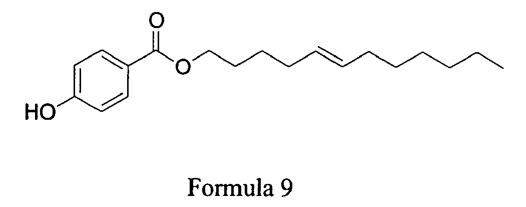 which is the 4-Hydroxy-benzoic acid dodec-5-enyl ester,

The invention also encompasses the use of these compounds, separately or in combination, or of the crude CH₂Cl₂ extract or fraction A or B, or of the compounds obtained by the process of the invention as preservative, more particularly in a cosmetic, pharmaceutical, food or toiletry composition.

Thus, the invention also encompasses a pharmaceutical composition comprising at least one paraben compound according to the invention or obtained by the process of the invention.

It also encompasses a cosmetic composition comprising at least one paraben compound according to the invention or obtained by the process of the invention.

It further encompasses a food composition comprising at least one paraben compound according to the invention or obtained by the process of the invention.

A toiletry composition comprising at least one paraben compound according to the invention or obtained by the process of the invention is also encompassed by the invention.

The invention also encompasses the use of the paraben compounds of the invention, separately or in combination, or of the crude CH₂Cl₂ extract or of fraction A or B, as antifouling agents.

## Claims

1. The *Microbulbifer* strain, named L4-N2, deposited at the CNCM under the registration number I-3714.

2. A process for manufacturing paraben compounds, **characterized in that** it comprises culturing the *Microbulbifer* strain of claim 1 and recovering the paraben compounds from the culture.

3. The process of claim 2, **characterized in that** said *Microbulbifer* strain is grown at a temperature of from 12 to 20°C, and the paraben compounds are recovered from the culture in the stationary phase.

4. The process for manufacturing paraben compounds according to claim 1 or 2, **characterized in that** paraben compounds are recovered from the culture in the stationary phase by an extraction with CH₂Cl₂ or ethyl acetate, to obtain a crude extract.

5. The process for manufacturing paraben compounds according to claim 4, **characterized in that** it further comprises a step of extraction by chromatography of the CH₂Cl₂ crude extract, the eluent used during the chromatography being cyclohexane to which increasing amounts of ethyl acetate are added, and recovering the fraction eluted with 80% by volume of cyclohexane and 20% by volume of ethylacetate and the fraction eluted with 70% by volume of cyclohexane and 30 % by volume of ethylacetate.

6. A process for manufacturing paraben compounds according to claim 5, **characterized in that** it further comprises the step of isolation of each paraben compound by reverse phase HPLC.

7. A crude extract comprising a mixture of paraben compounds obtainable by a process comprising culturing the *Microbulbifer* strain of claim 1 at a temperature of from 12 to 20°C, separating cells from the culture medium, in the stationary phase, by centrifugation, pouring the culture supernatant in CH₂Cl₂ or ethyl acetate and incubating at least 12 hours, and mechanically lysing the bacterial pellet and incubating the lysed bacterial pellet in CH₂Cl₂ or ethyl acetate during at least 12 hours to extract the mixture of paraben compounds.

8. A fraction comprising a mixture of paraben compounds obtainable by a process comprising the following steps:
a) culturing the *Microbulbifer* strain of claim 1 at a temperature of from 12 to 20°C,
b) separating cells from the culture medium, in the stationary phase, by centrifugation, thereby obtaining a supernatant and a bacterial pellet,
c) pouring in CH₂Cl₂ the supernatant obtained in step b) and incubating it during at least 12 hours,
d) mechanically lysing the bacterial pellet obtained in step b) and incubating the lysed bacterial pellet in CH₂Cl₂ during at least 12 hours, thereby obtaining a second CH₂Cl₂ extract,
e) mixing the CH₂Cl₂ extracts obtained in step c) and d), and chromatographiing the obtained mixture on a silicagel column using cyclohexane with increasing amounts of ethyl acetate as eluent, and
f) recovering the fraction eluted with 70% by volume of cyclohexane and 30% by volume of ethyl acetate.

9. A fraction comprising a mixture of paraben compounds obtainable by a process comprising the following steps:
a) culturing the *Microbulbifer* strain of claim 1 at a temperature of from 12 to 20°C,
b) separating cells from the culture medium, in the stationary phase, by centrifugation, thereby obtaining a supernatant and a bacterial pellet,
c) pouring in CH₂Cl₂ the supernatant obtained in step b) and incubating it during at least 12 hours,
d) mechanically lysing the bacterial pellet obtained in step b) and incubating the lysed bacterial pellet in CH₂Cl₂ during at least 12 hours, thereby obtaining a second CH₂Cl₂ extract,
e) mixing the CH₂Cl₂ extracts obtained in step c) and d), and chromatographiing the obtained mixture on a silicagel column using cyclohexane with increasing amounts of ethyl acetate as eluent, and
f) recovering the fraction eluted with 80% by volume of cyclohexane and 20% by volume of ethyl acetate.

10. A paraben compound which is the 4-hydroxy-benzoic acid 3-hydroxy-decyl ester having the following formula 5:

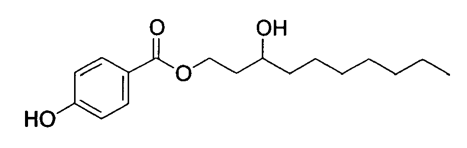

11. A paraben compound which is the 4-hydroxy-benzoic acid methyl-decyl ester having the following formula 6:

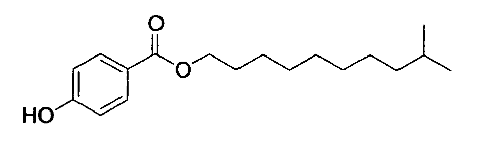

12. A paraben compound which is the 4-hydroxy-benzoic acid 3-hydroxy-methyl-decyl ester having the following formula 7:

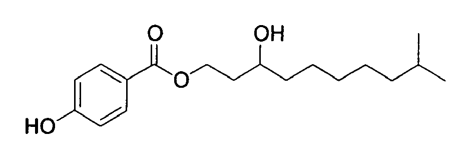

13. A paraben compound which is the 4-hydroxy-benzoic acid dodec-5-enyl ester having the following formula 9:

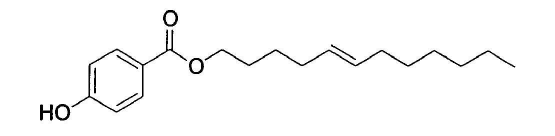

14. The use of the crude extract according to claim 7, or of the fraction according to claim 8 or 9, or of at least one paraben compound according to any one of claims 10-13, or of at least one paraben compound obtained by the process of any one of claims 2-6, as a preservative agent.

15. A pharmaceutical composition, **characterized in that** it comprises the crude extract according to claim 7, or the fraction according to claim 8 or 9, or at least one paraben compound according to any one of claims 10-13, or at least one paraben compound obtained by the process of any one of claims 2-6.

16. A cosmetic composition **characterized in that** it comprises the crude extract according to claim 7, or the fraction according to claim 8 or 9, or at least one paraben compound according to any one of claims 10-13, or at least one paraben compound obtained by the process of any one of claims 2-6.

17. A food composition **characterized in that** it comprises the crude extract according to claim 7, or the fraction according to claim 8 or 9, or at least one paraben compound according to any one of claims 10-13, or at least one paraben compound obtained by the process of any one of claims 2-6.

18. A toiletry composition **characterized in that** it comprises the crude extract according to claim 7, or the fraction according to claim 8 or 9, or at least one paraben compound according to any one of claims 10-13, or at least one paraben compound obtained by the process of any one of claims 2-6.

19. A detergent composition **characterized in that** it comprises the crude extract according to claim 7, or the fraction according to claim 8 or 9, or at least one paraben compound according to any one of claims 10-13, or at least one paraben compound obtained by the process of any one of claims 2-6.

20. A home care product composition **characterized in that** it comprises the crude extract according to claim 7, or the fraction according to claim 8 or 9, or at least one paraben compound according to any one of claims 10-13, or at least one paraben compound obtained by the process of any one of claims 2-6.

21. A sanitizing and/or disinfectant composition **characterized in that** it comprises the crude extract according to claim 7, or the fraction according to claim 8 or 9, or at least one paraben compound according to any one of claims 10-13, or at least one paraben compound obtained by the process of any one of claims 2-6.

22. The use of the crude extract according to claim 7, or of the fraction according to claim 8 or 9, or of at least one paraben compound according to any one of claims 10-13, or of at least one paraben compound obtained by the process of any one of claims 2-6, as antifouling agent.
